# EUROPÄISCHE PATENTSCHRIFT

(11) **EP 1 130 402 B1**
(45) Veröffentlichungstag und Bekanntmachung des Hinweises auf die Patenterteilung: **01.03.2006**
(21) Anmeldenummer: 00125835.9
(22) Anmeldetag: 25.11.2000
(51) Int. Cl.: G01N 33/86, G01N 33/96

(54) **Gebrauchsfertiges langzeitstabiles Ristocetin Cofactor Testreagenz**
Ready-to-use long term stable ristocetin co-factor test reagent
Prête à l'emploi réactif d'essai de ristocétine cofacteur avec stabilité à long terme

(30) Priorität: 29.12.1999 DE 19964109
(43) Veröffentlichungstag der Anmeldung: 05.09.2001
(73) Patentinhaber: Dade Behring Marburg GmbH, 35001 Marburg (DE)
(72) Erfinder: Patzke, Jürgen, Dr., 35039 Marburg (DE); Braun, Konrad, 35085 Ebsdorfergrund (DE)

(56) Entgegenhaltungen:
- WO-A-99/55346
- DE-A- 3 141 894
- US-A- 5 378 601
- US-A- 5 919 614
- THOMAS KATHY B ET AL: "Parallel determination of Von Willebrand factor-ristocetin and botrocetin cofactors" THROMBOSIS RESEARCH, Bd. 75, Nr. 4, 1994, Seiten 401-408, XP009023126 ISSN: 0049-3848

## Beschreibung

Die vorliegende Erfindung betrifft ein gebrauchsfertiges langzeitstabiles Ristocetin-Cofaktor Testreagenz zur Verwendung in Gerinnungstests.

Das von-Willebrand -Jürgens-Syndrom ist die am häufigsten vorkommende Blutungsneigung. Bei 1% der Bevölkerung ist das von Willebrand-Protein (vWF) quantitativ vermindert oder seine Funktion ist gestört. Zur Diagnose der Krankheit und ihrer unterschiedlichen Formen werden verschiedene Teste durchgeführt. Als wichtigster Screening-Test hat sich der Ristocetin-Kofaktor Test (vWF:RCo) etabliert (Rodeghiero F. et al., Thromb.Haemost (1990) 64, Seiten 349-352). Nach Untersuchungen von Rodeghiero (1990) weist er eine Sensitivität von 50 % auf, während der zweitwichtigste Test, die vWF-Antigen-Bestimmung (vWF:Ag), nur bei 32% liegt.

Ristocetin ist ein Antibiotikum, das auf Grund seiner starken Nebenwirkungen schnell vom Markt zurückgezogen wurde. Bereits im Jahr 1960 entdeckten Gangarosa et al. (Gangarosa EJ et a., Arch.Intern.Med. (1960), Seiten 83-89), daß Ristocetin in vitro die Aggregation von Kaninchen-Thrombozyten bewirkt. Auch humane Plättchen in Plättchen-reichem Plasma (PRP) aggregieren in Gegenwart von Ristocetin, allerdings nicht bei Patienten mit von-Willebrand-Syndrom (Howard and Firkin, Thromb.Diath.Haemorrh (1971), 26, Seiten 362-369). Darauf aufbauend entwickelten Weiss et al. (Weiss HJ et al., J.Clin.Invest. (1973) 52, Seiten 2708-2716) einen quantitativen Test mit gewaschenen Thrombozyten von Normalspendern. Die gewaschenen Thrombozyten sind allerdings nur wenige Stunden verwendbar. Es sind auch fixierte Plättchen eingesetzt worden, die den Vorteil haben, ihre Aktivität über eine längere Zeit zu erhalten, so daß die aufwendig Aufarbeitung von frischem PRP entfällt (Allain JP et al., J.Lab.Clin.Med. (1975), 85, Seiten 318-328.

Das Prinzip des Ristocetin-Kofaktor-Tests ähnelt der In vivo-Funktion des Proteins, weswegen der Test häufig als Aktivitätstest bezeichnet wird. Durch die Zugabe von Ristocetin in einen Testansatz mit Thrombozyten und von Willebrand-Faktor wird die Bindung des vWF-Moleküls an den GPIb/V/IX-Rezeptor und damit die Thrombozyten-Agglutination ausgelöst. Die genaue Wirkungsweise ist noch nicht bekannt. Offensichtlich bindet Ristocetin an vWF und es wird angenommen, daß eine entscheidende Reaktion die, durch die Bindung von Ristocetin, hervorgerufene Konformationsänderung des vWF-Muleküls ist, so daß dieses dann seinerseits an GPIb/V/IX binden kann. Dieser Vorgang wäre vergleichbar mit der Konformationsänderung durch die Bindung an das Subendothel, die den vW-Faktor in vivo (besonders bei hohen Scherkräften) bindungsfähig machen.

Über die Stabilität von fixierten Thrombozyten in Bezug auf die vWF:Rco-Aktivität bei flüssiger Lagerung gibt es Angaben, die zwischen 1 Monat (Allain et al., J. Lab. Clin. Med. 1975, 85, Seiten 318-328) und bis zu 6 Monaten schwanken (Thomas et al. (1994). Thromb.Res. 75, 4, Seiten 401-408).

Für den kommerziellen Einsatz ist allerdings eine Stabilität von mindestens 10, besser noch mindestens 14 Monaten notwendig.

Eine wesentliche Verbesserung der Stabilität konnte bisher nur durch eine Lyophilisation der Thrombozyten erreicht werden (US 4,145,185; DE 3141894A1; WO 99/55346) oder durch Kryokonservierung (US 5,378,601; US 5,919,614). In dem US Patent Nr. 4,145,185 wird dabei der Zusatz eines Schlangengiftes beschrieben. In der DE 3141894 wird die Verwendung von einem Gerbstoff, einem Serin-Proteinasen Inhibitor und Ristocetin A beschrieben. In der internationalen Anmeldung WO 99/55346 wird eine Methode beschrieben, bei der die Thrombozyten durch Diafiltration und Fixierung stabilisiert werden. In dem US Patent Nr. 5,378,601 wird eine Methode beschrieben, bei der die Thrombozyten durch Zusatz von Apyrase und eines Antioxidans stabilisiert werden. In dem US Patent Nr. 5,919,614 wird eine Methode beschrieben, bei der die Thrombozyten bis zur Verwendung in einer Lösung aufbewahrt werden, die eine Reihe verschiedener Inhibitoren der Plättchenfunktion enthält. Die zur Zeit kommerziell erhältlichen Thrombozyten-Reagenzien für den Ristocetin-Kofaktor-Test sind alle lyophilisiert. Besonders bei der Verwendung eines solchen, lyophilisierten Reagenzes in einem voll- oder tellautomatisierten Analysengerät ist es von Nachteil, dass das Produkt nach Öffnen nicht direkt einsetzbar ist; das Lyophilisat muß zunächst rekonstituiert werden. Darüber hinaus ist die Lyophilisation ein teures Verfahren, das zudem noch, alleine schon wegen der zusätzlichen Verfahrensschritte, sowohl bei der Herstellung als auch bei der Verwendung, die Gefahr zusätzlicher Fehler und Schädigungen der Thrombozyten in sich birgt. Bedingt durch das Herstellungsverfahren ist bei lyophilisierten Reagenzien oft eine relativ große Abweichung der Ergebnisse innerhalb einer Produktionscharge von Fläschchen zu Fläschchen zu beobachten.

Die der vorliegenden Erfindung zugrunde liegende Aufgabe bestand daher darin, ein Verfahren für den Ristocetin-Cofaktor-Test unter Verwendung einer Flüssigformulierung eines Thombozytenreagenzes bereit zu stellen, das eine Verwendung des Flüssigreagenzes auch nach einer Lagerzeit von deutlich mehr als 6 Monaten, bevorzugt mindestens 10 Monaten, besonders bevorzugt mindestens 14 Monate ermöglicht, d.h,, dass bei Einsatz des Reagenzes in einem Test auch nach dieser Lagerzeit die Spezifikationen des Testes wie z.B. Meßbereich und Präzision erfüllt werden.

Ein solches Reagenz ist für das Gerinnungslabor außerordentlich vorteilhaft, da es die Vorteile der leichten Handhabbarkeit (Gebrauchsfertigkeit) mit den Vorteilen der hohen Sensitivität und guten Reproduzierbarkeit kombiniert. Weitere bevorzugte Ausführungsformen der Erfindung sind auch den Patentansprüchen zu entnehmen.

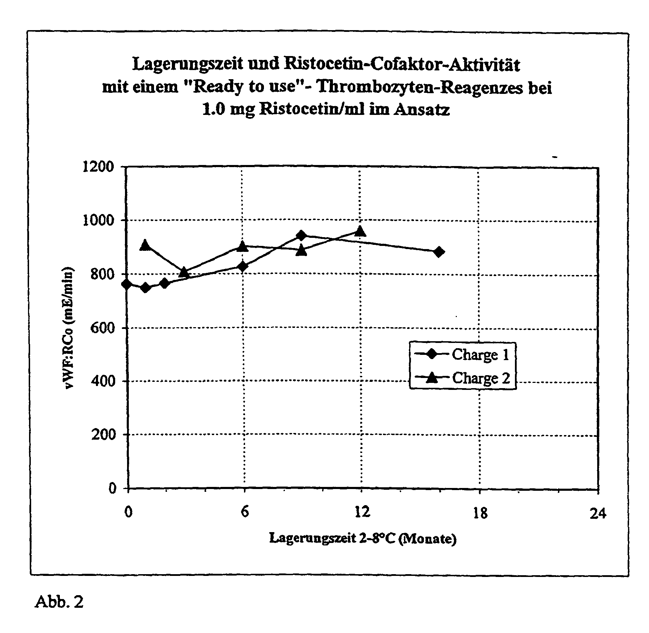

Überraschenderweise wurde nun gefunden, daß in einer ready-to-use (etwa 600.000 Thrombocyten/ µl) Flüssigpräparation eine Stabilität von 12 Monaten und mehr erreichbar ist, wenn die Thrombocyten bei der Aufarbeitung mit einem Serin-Protease Hemmstoffe (vorugsweise Diisopropylfluorphosphat) behandelt werden, fixiert werden und in einer wässrigen, gepufferten Lösung suspendiert werden, die kein Ristocetin und kein Schlangengift enthält (Abb. 2)

Verbessert werden kann die Lagerungsstabilität u.a. durch die Zugabe von Neutralproteinen, wie Albuminen, insbesondere humanes oder bovines Serumalbumin. Bei ausreichender Albuminkonzentration (größer 0,2%, vorzugsweise 2%) kann auch Ristocetin zu der Flüssigpräparation hinzugefügt werden. Wesentlich verbessert werden kann die Lagerungsstabilität, auch in Gegenwart von Ristocetin, durch die Behandlung mit oder die Zugabe eines Isothiazolons, wie zum Beispiel Kathon®, bevorzugterweise Kathon® CG (Rohm & Haas, Philadelphia, USA), oder ProClin® , oder eines anderen bakeriziden oder bakteriostatischen Stoffes. Vorteilhafterweise werden diese Verbindungen in einer Konzentration (vol/vol) von 0,1 bis 2%, besonders vorteilhafterweise 0,5 bis 1,4%, ganz besonders vorteilhafterweise 0,8 bis 1,2% im Reagenz eingesetzt.

Weitere, dem Fachmann an sich bekannte Zusätze, wie sie z. B. in der DE 3141894 beschrieben sind, wirken sich zusätzlich vorteilhaft auf die Langzeitstabilität bei flüssiger Lagerung aus.

Sofern nicht anders angegeben, bezieht sich der Begriff Lagerung u.ä. auf die Lagerung bei 2-8°C (Kühlschrank) und der Begriff Stabilität u.ä. auf die Bestimmung des vWF:RCo Tests (Thomas et al. (1994)).

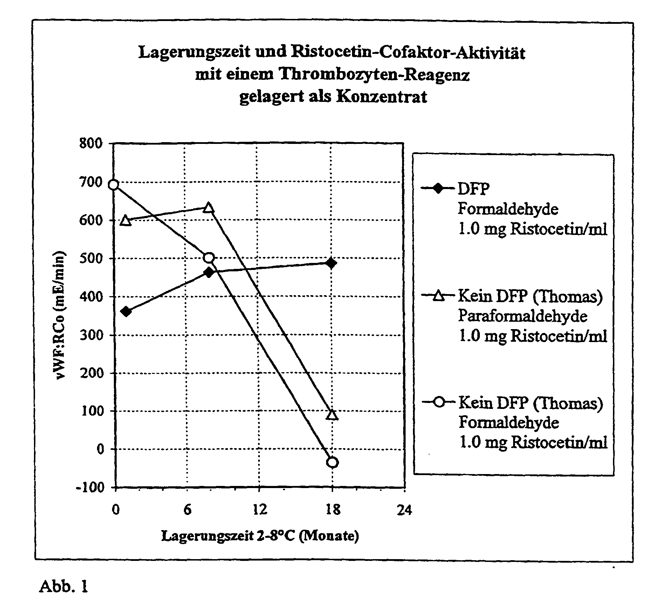

Die Lagerung des Reagenzes mit einer Konzentration von Thrombozyten, die das Reagenz ohne Verdünnung direkt für den Test einsetzbar machen (Ready-to-use) läßt nach dem bekannten Stand der Technik eine schlechtere Haltbarkeit erwarten als die Lagerung als Konzentrat. Ein Konzentrat enthält typischerweise 5-15 Mio. Thrombozyten/µl. Tatsächlich zeigt das erfindungsgemäße Reagenz auch bei einem Reagenz mit 600.000 Thrombozyten/µl eine Haltbarkeit des Reagenzes von mehr als 12 Monaten (Abb. 1 und Abb. 2).

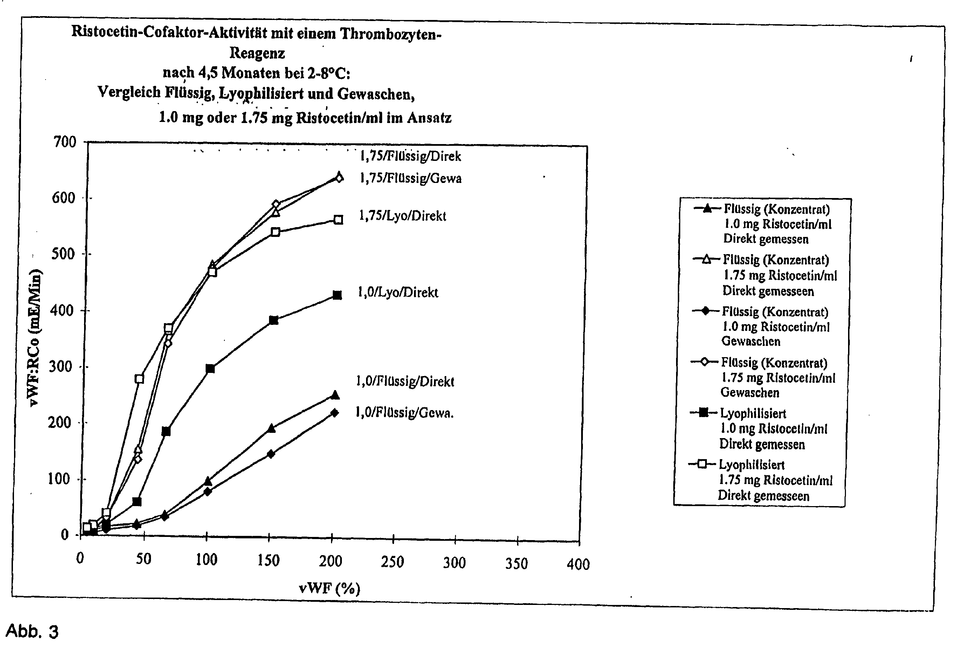

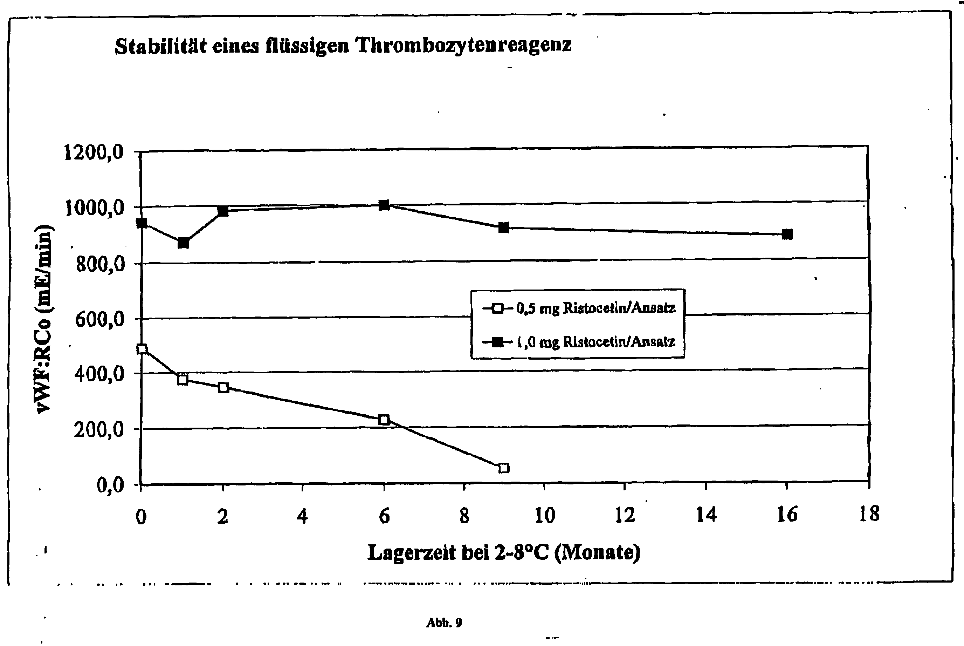

Unabhängig von der Art des Reagenzes wurde darüber hinaus gefunden, daß die Erhöhung der Ristocetinkonzentration unabhängig davon wieviel Albumin im Reagenz oder Testansatz vorliegt im Testansatz auf über 1,25 mg/ml einen positiven Einfluß auf die Verwendbarkeit von lange gelagertem Thrombozyten-Reagenz haben kann. Ein Reagenz, das bei 1,0 mg/ml Ristocetin im Testansatz nicht mehr aktiv genug ist, kann bei Verwendung von z.B. 1,5 mg/ml ausreichende Aktivität zeigen und auch im Laufe der Reagenzlaufzeit keinen Aktivitätsverlust zeigen (Abb. 3,4 und 9).

Ein Beispiel für diesen positiven Effekt der erhöhten Ristocetinkonzentration im Testansatz ist die Lagerung von einem Thrombozytenkonzentrat (100 ml) in einem Erlenmeyerkolben (250 ml), der zur Reagenzentnahme für die Meßzeitpunkte geöffnet und wieder verschlossen wurde. Das häufige Öffnen bewirkt einen starken Luftkontakt und eine hohe Kontaminationsgefahr. Bei Verwendung der üblichen Ristocetinkonzentration im Testansatz von 1.0 mg/ml ist nach 4,5 Monaten Lagerzeit die Reaktivität der Thrombozyten, das heißt die Ristocetin-Kofaktor-Aktvitität, stark gesunken. Es wurde in diesem Fall kein Ausgangswert bestimmt, aber die Aktivität des Konzentrates kann auch mit der Aktivität von lyophilisierten Thrombozyten (der gleichen Charge) verglichen werden, die bekanntermaßen sehr stabil sind (Abb. 3). Beim Vergleich von Flüssiglagerung und Gefriertrocknung ergibt sich nach 4 ½ Monaten Lagerung ein starker Reaktivitätsverlust, wenn die Ristocetinkonzentration im Ansatz 1,0 mg/ml beträgt und es ergibt sich dagegen kein wesentlicher Reaktivitätsunterschied, wenn die Ristocetin-Konzentration im Ansatz 1,75 mg/ml beträgt (Abb. 3). Der schädigende Einfluß der Lagerung im Erlenmeyerkolben (Luftkontakt, Verunreinigung, Alterung) wird also durch die höhere Ristocetinkonzentration kompensiert. Dieser Lagerungseffekt ist vermutlich auch die Erklärung für den relativ starken Reaktivitätsabfall dieser Messung gegenüber den Ergebnissen aus dem in Abb. 2 dargestellten Versuch.

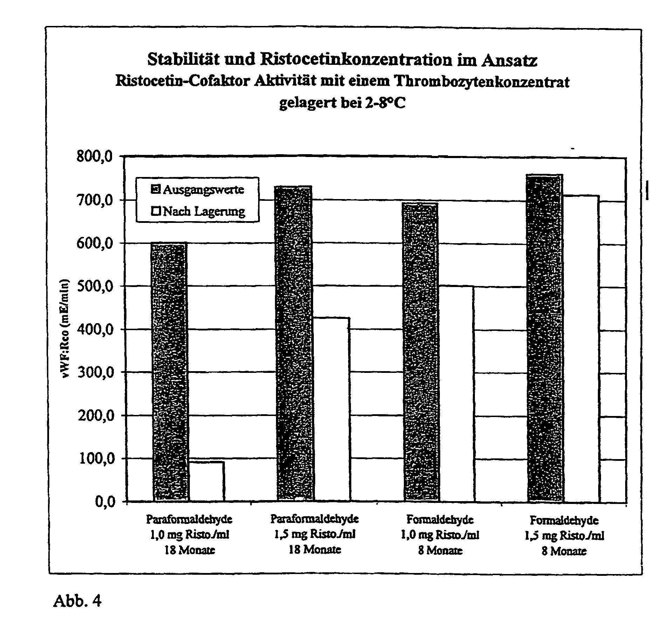

Ein weiteres Beispiel für den positiven Effekt der erhöhten Ristocetinkonzentration im Testansatz ist die Reagenzherstellung nach Thomas et al. (1994), die zu einem 6 Monate stabilem Reagenz führen soll. Nach 18 Monaten ist die Aktivität erwartungsgemäß extrem stark abgefallen, wenn im Testansatz 1,0 mg Ristocetin verwendet werden. Bei 1,5 mg Ristocetin im Testansatz ist die Reaktivität bei weitem nicht so stark abgefallen wird (Abb. 4, Paraformaldehyd als Fixierungsmittel). Auch bei der Fixierung mit Formalin und 8 Monaten Lagerungsdauer ist der Abfall der Reaktivität bei 1,5 mg Ristocetin im Testansatz nicht so stark (Abb. 4).

Die erhöhte Ristocetin-Konzentration bewirkt also eine Kompensation von Alterungsprozessen. Diese Alterungsprozesse können bei unvorteilhafter Lagerung auch bei vorheriger Behandlung mit DFP auftreten (Abb. 3) oder bei einem Reagenz nach dem Stand der Technik (Thomas et al., 1994,) Abb. 4)).

Vorteilhafterweise kann eine erhöhte Ristocetin-Konzentration im Testansatz auch in Verbindung mit dem erfindungsgemäßen Reagenz ausgewählt werden.

Bevorzugterweise beträgt die Ristocetinkonzentration im Testansatz 1 bis 2,4 mg/ml, besonders bevorzugterweise 1,25 bis 2 mg/ml.

Die Ursache für den erhöhten Bedarf an Ristocetin könnte auch die im Laufe der Zeit erhöhte Proteinkonzentration im Puffer sein, da bekannt ist, daß Ristocetin an verschiedene Proteine bindet und eine erhöhte Proteinkonzentration durch mehr Ristocetin kompensiert werden kann (Stibbe und Kirby, Thromb. Res. (1976), 8, Seiten 151-165). Daher wurden 4 ½ Monate gelagerte Thrombozyten gewaschen und in frischem Puffer (der wieder Ristocetin enthält) aufgenommen. Die Aktivität änderte sich durch das Waschen weder bei 1.0 mg/ml noch bei 1,75 mg/ml Ristocetin im Testansatz . Daraus ist der Schluß zu ziehen, daß der Alterungsprozeß sich tatsächlich auf die Thrombozyten selbst auswirkt (Abb. 3). Offensichtlich bewirkt das Altem ein verminderte Sensitivität gegenüber der Wirkung von Ristocetin. Dies ist insofern überraschend, als bis jetzt die Hauptwirkung des Ristocetins im Ristocetin-Kofaktor Test in einer Art von Aktivierung des von Willebrand Proteins gesehen wid, welche das Protein bindungsfähig für den GPlb-Rezeptor macht. Wenn gealterte Thrombozyten aber mehr Ristocetin für die gleiche Agglutinationsaktivität benötigen, ist offensichtlich die direkte Wechselwirkung von Ristocetin und Thrombozyten wichtiger für den Ristocetin-Kofaktor Test als bisher angenommen wurde.

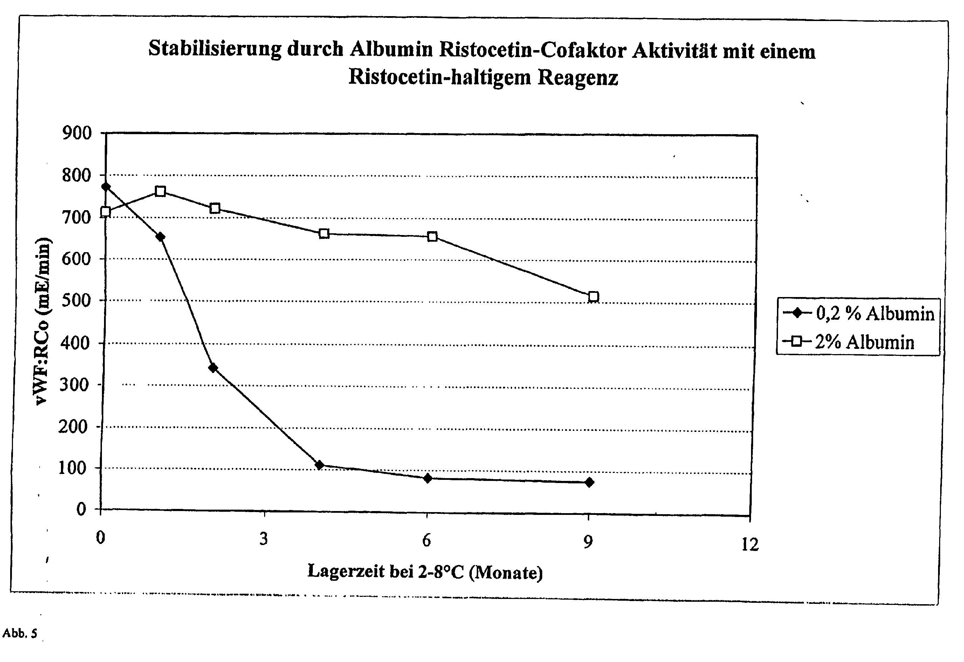

Fügt man dem Thrombozyten-Reagenz bereits während der Lagerung Ristocetin hinzu, so ist das Reagenz weniger stabil als ohne Ristocetin. Allerdings kann die Stabilität durch die Zugabe eines Neutralproteins in einer Konzentration von mehr als 1 % , bevorzugterweise Albumin, ganz bevorzugterweise humanes oder bovines Albumin, wesentlich verbessert werden. So ist die Stabilität bei 2% Albumin besser als bei 0.2 % Albumin (Abb. 5).

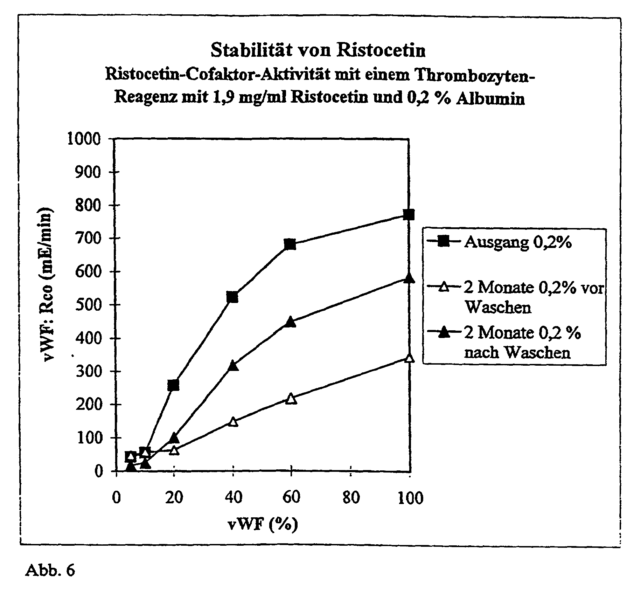

Wäscht man Thrombozyten, die mit 0,2% Albumin und Ristocetin gelagert wurden mit frischem Puffer, der ebenfalls 0,2% Albumin und Ristocetin enthält, so wird die Reaktivität wieder stärker, kommt aber nicht auf das Ausgangsniveau zurück (Abb. 6, linkes Diagramm). Daraus kann geschlossen werden, dass die Thrombozyten selbst also gealtert sind .

Aus der Literatur ist bekannt, daß Ristocetin Fibrinogen und andere Proteine präzipitiert. Diese Reaktion ist bereits bei 0.5 mg/ml erkennbar und bei 2.0 mg/ml ist alles Fibrinogen im Plasma präzipitiert, während die anderen Plasmaproteine noch nicht betroffen sind (Howard and Firkin, Thromb. Diath.Haemorrh. (1971), 26, Seiten 362-369).

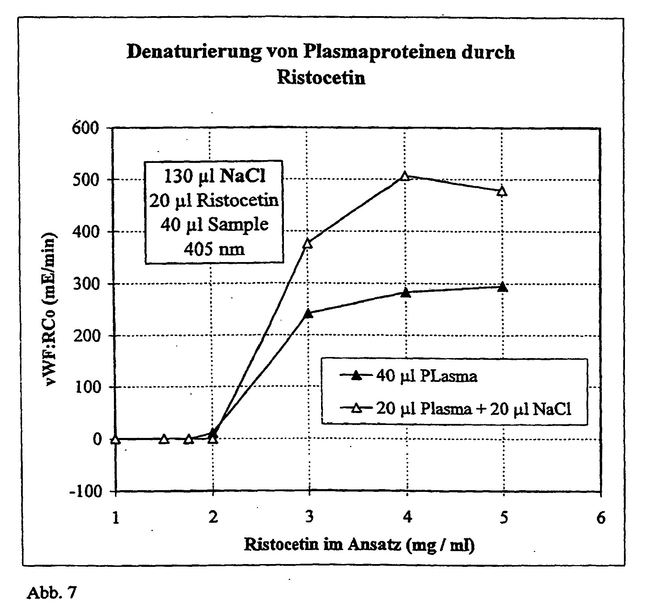

Es zeigte sich, daß im erfindungsgemäßen Verfahren bis zu einer Ristocetin-Konzentration im Ansatz von 1.75 mg/ml (40 µl Probe in 190 µl Testansatz), bzw. 2.0 mg/ml (20 µl Probe in 190 µl Testansatz) keine störende Proteinpräzipitation auftritt (Abb. 7).

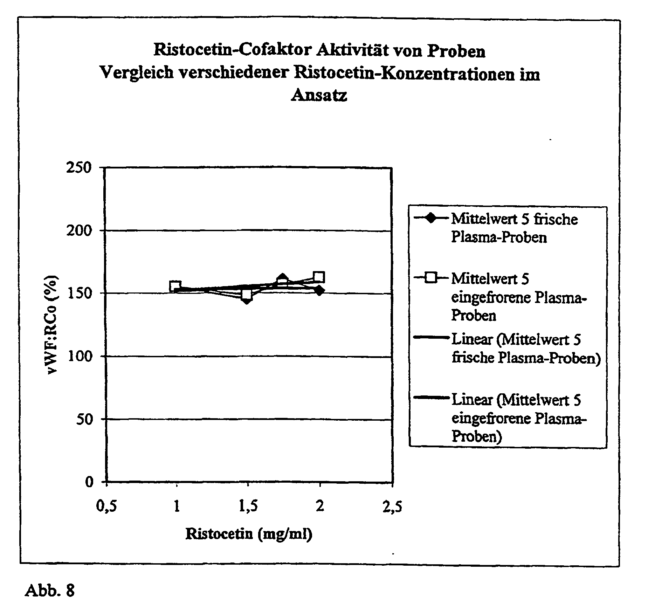

Bei der Messung von Proben im vWF:RCo-Test zeigten sich keinen signifikanten Ergebnisunterschiede bei Ristocetinkonzentrationen bis zu 2.0 mg/ml (Abb. 8). Die übliche Konzentration von Ristocetin im vWF:RCo-Testansatz mit fixierten Thrombozyten beträgt 1 mg/ml. (Thomas et al. 1994: 1.0 mg/ml).

### Beschreibung der Abbildungen

### Abb.1

Stabilität von Thrombozyten-Konzentrat-Reagenz, das entweder mit DFP wie im Beispiel 1 oder ohne DFP wie bei Thomas et al. (1994), Thromb.Res. 75, 4, Seiten 401-408) hergestellt wurde.

### Abb. 2

Stabilität eines "Ready to use"-Thrombozyten-Reagenzes mit 600.000 Thrombozyten/µl.

### Abb. 3

Aktivität von Thrombozyten-Konzentrat-Reagenz nach 4,5 Monaten Lagerung bei 1,75 und 1,0 mg/ml Ristocetin im Testansatz.

### Abb. 4

Auswirkung von der Ristocetin-Konzentration im Testansatz auf die Aktivität des Reagenzes nach 18 bzw. 8 Monaten Lagerung. Die Paraformaldehyd-Aufarbeitung erfolgte wie bei Thomas et al. (1994), Thromb.Res. 75, 4, Seiten 401-408), die Formaldehyd-Aufarbeitung wie im Beispiel 1. Die Ristocetin-Konzentration bezieht sich auf den Testansatz.

### Abb. 5

Stabilität eines Ristocetin-haltigen Thrombozyten-Reagenzes mit 0,2% und 2% Albumin.

### Abb. 6

Abfall der Reaktivität bei verschiedenen von Willebrand-Konzentrationen eines Ristocetin-haltigen Thrombozyten-Reagenzs mit 0,2% Albumin nach 2 Monaten. Nach dem Waschen der Thrombozyten mit frischen Puffer gleicher Zusammensetzung wie zu Beginn der Lagerung, steigt die Reaktivität wieder etwas an.

### Abb. 7

Präzipitation von Proteinen der Probe durch hohe Ristocetin-Konzentrationen.

### Abb. 8

Einfluß von hohen Ristocetin-Konzentrationen auf die Bestimmung der von Willebrand-Konzentration mit Hilfe des Ristocetin-Kofaktor-Tests.

### Abb. 9

Abfall der Reaktivität eines Thrombozyten-Reagenzes bei 0,5 und 1,0 mg/ml Ristocetin im Testansatz.

Die folgenden Beispiele erläutern die Erfindung.

### Beispiel 1

Thrombozyten wurden aus Vollblut durch Abzentrifugation der Erythrozyten isoliert. Dreimaliges Waschen der isolierten Thrombozyten erfolgte durch Aufnahme in Phosphatpuffer A (0,07 mol Phosphat/l, 8,5 g NaCl/L, 1,9 g EDTA/l) und Abzentrifugation; Einstellung der Thrombozytenzahl auf ca. 3 Mio./µl.

Nach Zugabe von Diisopropylfluorphosphat (DFP,4,5x10E-4 M) wurde 1 Stunde bei Raumtemperatur unter Rühren inkubiert. Nach zweimaligem Waschen durch Abzentrifugation und Wiederaufnahme in Phosphatpuffer B (0,15 mol Phosphat/l, 1,0 g EDTA/l, PH 6,5) wurde nach der Zugabe von Formaldehyd (Endkonzentration 4%) eine Inkubation von 44 Stunden bei 4°C angeschlossen. Die Thrombozyten wurden abzentrifugiert in Phosphatpuffer C (0,07 mol Phosphat/l, 8,5 g NaCl/L, 1,0 g EDTA/l, 1g Natriumazid/I) aufgenommen.

Anschließend erfolgte eine Dialyse der Thrombozytensuspension gegen das 20fache Volumen Phosphatpuffer C über ca. 24 Stunden. Danach wurde die Thrombozytenzahl durch Zugabe von Phosphatpuffer C eingestellt. Ferner wurden zugegeben: Saccharose (10g/l), Glycin (10 g/l), Glutaminat (16,7 g/l) und Humanalbumin (2g/l).

Anschliessend erfolgte die Abfüllung in 5 ml-Fläschchen mit luftdichtem Schraubverschluß.

Vor der Bestimmung (Messung der Extinkion in mE) wurde zu einer 5 ml Abfüllung 0,5 ml Ristocetinlösung (21 mg/ml) zugegeben, die Ristocetinkonzentration im Reagenz betrug somit 1,91 mg/ml. Der Testansatz setzte sich zusammen aus:

| | |
|---|---|
| 150 µl | Reagenz |
| 20 µl | Plasma und |
| 20 µl | isotonische NaCl Lösung (0,9%) |

Bestimmt wurde die Geschwindigkeit der Extinktionsabnahme (Vₘₐₓ). Die Ristocetinkonzentration im Testansatz betrug 1.5 mg/ml.

### Beispiel 2

Durchführung wie in Beispiel 1, allerdings wurde die Ristocetinkonzentration im Thrombozytenreagenz durch Zugabe vor der Messung auf 1.3 mg/ml eingestellt und somit betrug die Ristocetinkonzentration im Testansatz 1,0 mg/ml (Abb. 1,2 und 9).

### Beispiel 3

Durchführung wie in Beispiel 1, allerdings wurde die Ristocetinkonzentration im Thrombozytenreagenz vor der Messung auf 2.2 mg/ml eingestellt und somit die Ristocetinkonzentration im Testansatz 1,75 mg/ml (Abb. 3).

### Beispiel 4

Durchführung wie in Beispiel 1, allerdings wurde vor der Abfüllung in 5 ml-Fläschchen mit luftdichtem Schraubverschluß soviel Ristocetin hinzugegeben, dass die Endkonzentration im Reagenz 1,9 mg/ml betrug. Dies entspricht einer Ristocetinkonzentration im Testansatz 1,5 mg/ml (Abb. 5)

## Patentansprüche

1. Flüssigpräparation fixierter Thrombozyten mit einer Stabilität von mindestens 10 Monaten, bevorzugterweise 12 Monaten, bestehend aus einer Thrombozytensuspension, wobei die Thrombozyten bei der Aufarbeitung mit einem Serin-Protease Hemmstoff behandelt werden, fixiert werden und in einer wässrigen, bevorzugterweise gepufferten Lösung suspendiert werden, die kein Ristocetin und kein Schlangengift enthält.

2. Präparation gemäß Anspruch 1 die auch nach Lagerung im flüssigen Zustand über einen Zeitraum von mindestens 12 Monaten eine Reaktivität von mindestens 80 % des Ausgangswertes, gemessen im Ristocetin-Kofaktor Test, aufweist.

3. Präparation gemäß Anspruch 1 zur Bestimmung und Quantifizierung von von-Willebrand Faktor.

4. Präparation gemäß Anspruch 1 in einer ready-to-use (etwa 50.000-2.000.000 Thrombocyten/ µl) Präparation.

5. Präparation gemäß Anspruch 4 mit 200.000 -1.000.000 Thrombozyten/µl, bevorzugterweise etwa 600.000 Thrombozyten/µl.

6. Verwendung einer Präparation gemäß einem der Ansprüche 1 bis 5 im Ristocetin-Kofaktor Test, in dem die Ristocetin Konzentration 1 bis 2,4 mg/ml, bevorzugterweise 1,25 bis 2 mg/ml im Testansatz beträgt.

7. Testkit für einen Ristocetin-Kofaktor Test in dem eine Präparation gemäß einem der Ansprüche 1 bis 5 enthalten ist.

## Claims

1. A liquid preparation of fixed platelets having a stability of at least 10 months, preferably 12 months, and consisting of a platelet suspension, with the platelets, during the working-up, being treated with a serine protease inhibitor, being fixed and being suspended in an aqueous, preferably buffered solution which does not contain any ristocetin or snake venom.

2. A preparation as claimed in claim 1 which, even after having been stored in the liquid state for a period of at least 12 months, exhibits a reactivity of at least 80% of the starting value, as measured in a ristocetin cofactor test.

3. A preparation as claimed in claim 1 for measuring and quantifying von Willebrand factor.

4. A preparation as claimed in claim 1 in the form of a ready-to-use (approximately 50,000-2,000,000 platelets/µl) preparation.

5. A preparation as claimed in claim 4 containing 200,000-1,000,000 platelets/µl, preferably approximately 600,000/µl.

6. The use of a preparation as claimed in one of claims 1 to 5 in a ristocetin cofactor test in which the concentration of ristocetin in the test mixture is from 1 to 2.4 mg/ml, preferably from 1.25 to 2 mg/ml.

7. A test kit for a ristocetin cofactor test, which kit contains a preparation as claimed in one of claims 1 to 5.

## Revendications

1. Préparation liquide de thrombocytes fixés ayant une stabilité d'au moins 10 mois, de préférence de 12 mois, constituée d'une suspension de thrombocytes, dans laquelle les thrombocytes sont traités lors de leur traitement avec un inhibiteur de serine-protéase, fixés et mis en suspension dans une solution aqueuse, de préférence tamponnée, qui ne contient pas de ristocétine, ni de venin de serpent.

2. Préparation selon la revendication 1, qui présente, même après stockage à l'état liquide sur une période d'au moins 12 mois, une réactivité d'au moins 80% de la valeur initiale, mesurée dans le test de cofacteur ristocétine.

3. Préparation selon la revendication 1 pour déterminer et quantifier le facteur de von Willebrand.

4. Préparation selon la revendication 1 dans une préparation prête à l'emploi (approximativement 50 000 à 2 000 000 de thrombocytes/µl).

5. Préparation selon la revendication 4 avec 200 000 à 1 000 000 de thrombocytes/µl, de manière préférée approximativement 600 000 thrombocytes/µl.

6. Utilisation d'une préparation selon l'une quelconque des revendications 1 à 5, dans un test de cofacteur ristocétine, avec une concentration en ristocétine de 1 à 2,9 4 mg/ml, de préférence de 1, 25 à 2 mg/ml, dans la préparation de test.

7. Trousse de test pour un test de cofacteur ristocétine, qui contient une préparation selon l'une quelconque des revendications 1 à 5.
